# EUROPEAN PATENT SPECIFICATION

(11) **EP 4 051 149 B1**
(45) Date of publication and mention of the grant of the patent: **11.06.2025**
(21) Application number: 19809285.0
(22) Date of filing: 01.11.2019
(51) Int. Cl.: A61B 18/14, A61B 17/00

(54) **SYSTEM FOR USE IN SEALING A PORTION OF PLEURAL LAYERS TOGETHER**
SYSTEM ZUR VERWENDUNG BEIM VERSIEGELN EINES TEILS VON PLEURALEN SCHICHTEN MITEINANDER
SYSTÈME DESTINÉ À ÊTRE UTILISÉ POUR SCELLER UNE PARTIE DE COUCHES PLEURALES ENSEMBLE

(43) Date of publication of application: 07.09.2022
(73) Proprietor: Bard Peripheral Vascular, Inc., Franklin Lakes, NJ 07417 (US)
(72) Inventor: ADDISON, Jordan, Tempe, AZ 85281 (US); GLASPIE, Koltin, Tempe, AZ 85281 (US); STORM, Heather, Tempe, AZ 85281 (US)
(74) Representative: Hoffmann Eitle
(86) International application number: PCT/US2019/059308
(87) International publication number: WO 2021/086393

(56) References cited:
- US-A- 5 954 715
- US-A1- 2002 111 620
- US-A1- 2004 176 801
- US-A1- 2006 025 815
- US-A1- 2016 324 570
- US-B1- 6 770 070

## Description

### Cross-Reference To Related Applications

None.

### Technical Field

The present invention relates to a lung access procedure, such as a lung biopsy, and, more particularly, to a system for use in sealing a portion of pleural layers together.

### Background Art

Pneumothorax is a problematic complication of the lung biopsy procedure where air or fluid is allowed to pass into the pleural space as a result of the puncture of the parietal pleura and visceral pleura. Pneumothorax and, more so, pneumothorax requiring chest tube placement, are significant concerns for clinicians performing, and patients undergoing, percutaneous lung biopsies. The incidence of pneumothorax in patients undergoing percutaneous lung biopsy has been reported to be anywhere from 9-54%, with an average of around 15%. On average, 6.6% of all percutaneous lung biopsies result in pneumothorax requiring a chest tube to be placed, which results in an average hospital stay of 2.7 days.

Factors that increase the risk of pneumothorax include increased patient age, obstructive lung disease, increased depth of a lesion, multiple pleural passes, increased time that an access needle lies across the pleura, and traversal of a fissure. Pneumothorax may occur during or immediately after the procedure, which is why typically a CT scan of the region is performed following removal of the needle. Other, less common, complications of percutaneous lung biopsy include hemoptysis (coughing up blood), hemothorax (a type of pleural effusion in which blood accumulates in the pleural cavity), infection, and air embolism.

What is needed in the art is a system for use in sealing a portion of pleural layers together.

US 6,770,070 B1 discloses an apparatus for obtaining a lung biopsy with an apparatus capable of sealing tears within the lung and pleural space to reduce the risk of pneumothorax or pulmonary hemorrhage.

### Summary of Invention

The present invention provides a system for use in sealing a portion of pleural layers together and is directed to the system of claim 1. The dependent claims refer to preferred embodiments.

The disclosure is directed to a system for use in sealing a portion of pleural layers together. The system includes an electrical energy source, and an electrocautery probe electrically coupled to the electrical energy source. The electrocautery probe has a cannula shaft portion, a distal penetrating tip, and an intermediate portion interposed between the cannula shaft portion and distal penetrating tip. The electrocautery probe is configured to generate heat. A protein source is coupled to the intermediate portion of the electrocautery probe, wherein the protein source has a protein that is denatured by heat.

The invention according to claim 1 is directed to a system for use in sealing a portion of pleural layers together. The system may include a fluid source, an electrical energy source, a grounding pad, and a monopolar electrocautery probe. The fluid source is configured to deliver a sealing fluid, wherein the sealing fluid is heat-activated. The grounding pad is electrically coupled to the electrical energy source. The monopolar electrocautery probe is electrically coupled to the electrical energy source. The monopolar electrocautery probe and grounding pad cooperate to generate heat. The monopolar electrocautery probe has a cannula shaft portion, a distal penetrating tip, and an expandable portion interposed between the cannula shaft portion and distal penetrating tip. The cannula shaft portion has a cannula lumen coupled in fluid communication with the fluid source. The expandable portion is coupled in fluid communication with the fluid source via the cannula lumen. The expandable portion is configured to define a plurality of openings, and is configured such that the sealing fluid that is supplied by the fluid source exits the expandable portion through the plurality of openings to a location external to the monopolar electrocautery probe.

An advantage of the present invention is that the system allows the physician to create an airtight seal of the pleural layers prior to performing a lung procedure, such as a lung biopsy, thereby reducing the risk of pneumothorax during the procedure.

### Brief Description of Drawings

The above-mentioned and other features and advantages of this invention, and the manner of attaining them, will become more apparent and the invention will be better understood by reference to the following description of an embodiment of the invention taken in conjunction with the accompanying drawings, wherein:
Fig. 1 is a diagrammatic representation of a system for sealing a portion of the pleural layers together in a patient, in accordance with an aspect of the present invention;
Fig. 2 is a perspective view of a monopolar electrocautery device of the system of Fig. 1;
Fig. 3 is a block diagram of the system of Fig. 1;
Fig. 4 is a diagrammatic side view of a portion of the monopolar electrocautery device of Fig. 2, depicting a fluid source for delivering a sealing fluid to the cannula lumen of the monopolar electrocautery probe;
Fig. 5 is a perspective view of a portion of the monopolar electrocautery probe of Fig. 2, with the expandable portion in a collapsed state;
Fig. 6 is a perspective view of a portion of the monopolar electrocautery probe of Fig. 2, with the expandable portion in an expanded state;
Fig. 7 is a side view of the portion of the monopolar electrocautery probe of Fig. 6, showing an expansion member that is representative of each of the plurality of expansion members of the expandable portion, and with the remainder of the individual members of the plurality of expansion members broken away for clarity;
Fig. 8 is a perspective view of a variation of the embodiment of Figs. 1-7, which includes a coating over an intermediate portion of the monopolar electrocautery probe;
Fig. 9 depicts a section view of a portion of a chest wall and lung of a patient, and shows the expandable portion of the monopolar electrocautery probe in an expanded state to aid in pulling the pleural layers together; and
Figs. 10A and 10B depict a flowchart of a method of using the system of Fig. 1 for use in a lung access procedure to aid in preventing pneumothorax.

Corresponding reference characters indicate corresponding parts throughout the several views. The exemplifications set out herein illustrate at least one embodiment of the invention, and such exemplifications are not to be construed as limiting the scope of the invention in any manner.

### Description of Embodiments

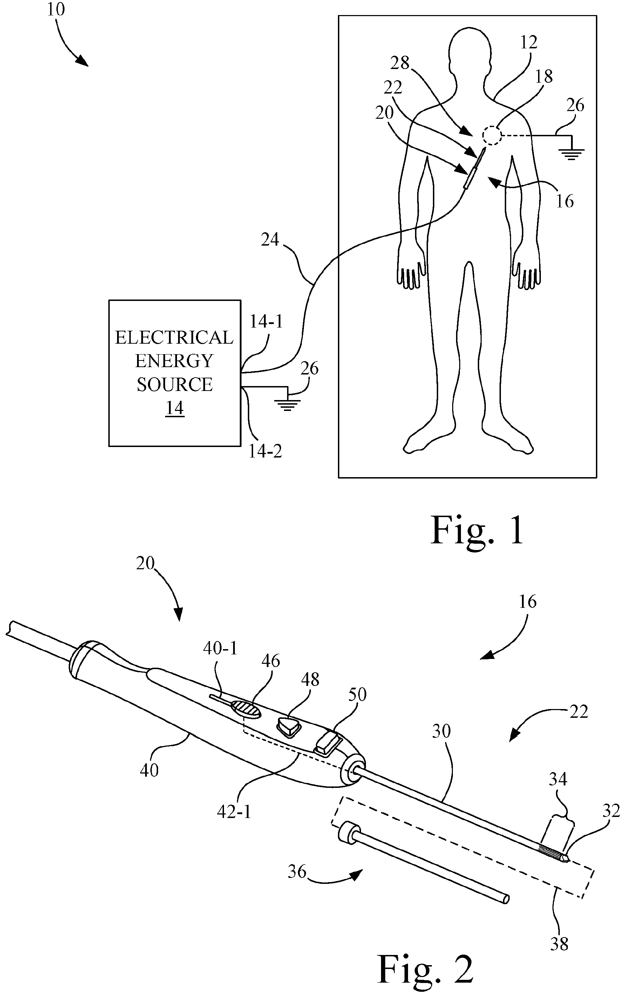

Referring now to the drawings, and more particularly to Fig. 1, there is shown a schematic diagram of an example of a system 10 for sealing a portion of the pleural layers in a lung procedure performed on a patient 12. In the present embodiment, system 10 generally includes an electrical energy source 14, a monopolar electrocautery device 16, and a grounding pad 18. Monopolar electrocautery device 16 includes a handpiece 20 connected to a monopolar electrocautery probe 22.

In the present embodiment, electrical energy source 14 may be, for example, an electrosurgical radio frequency (RF) generator. In the present embodiment, electrical energy source 14 includes a first RF output 14-1 and a second RF output 14-2.

First RF output 14-1 of electrical energy source 14 is electrically coupled to monopolar electrocautery device 16 via a connector cable 24. Connector cable 24 may be, for example, a multi-conductor cable that includes electrical conductors that supply control signals from handpiece 20 to electrical energy source 14 to control a power output of electrical energy source 14, and includes conductors (e.g., a shielded cable, such as an electrical coaxial cable) to supply electrical RF power signals to monopolar electrocautery probe 22 of monopolar electrocautery device 16. Accordingly, monopolar electrocautery probe 22 is electrically coupled to first RF output 14-1 of electrical energy source 14 via connector cable 24.

Second RF output 14-2 of electrical energy source 14 is electrically coupled to grounding pad 18 via a ground path 26. Grounding pad 18 is configured for contact with the patient 12, as is known in the art. It is contemplated that the ground path 26 between electrical energy source 14 and grounding pad 18 may be in the form of a shielded cable, such as an electrical coaxial cable.

Monopolar electrocautery probe 22 and grounding pad 18 form an RF circuit 28, wherein monopolar electrocautery probe 22 serves as a primary electrosurgical electrode and grounding pad 18 serves as a return electrode. Monopolar electrocautery probe 22 includes a cannula shaft portion 30, a distal penetrating tip 32, and an intermediate portion 34 interposed between the cannula shaft portion 30 and distal penetrating tip 32.

Monopolar electrocautery probe 22 and grounding pad 18 cooperate to generate heat when energized with RF energy. More particularly, electrical energy source 14 includes circuitry, as is known in the art, for generating an RF output signal having an RF frequency which may be, for example, in a range of 1.0 megahertz (MHz) to 10.0 MHz. The RF output signal generated by electrical energy source 14 is delivered to monopolar electrocautery probe 22 and grounding pad 18, so as to generate a heating effect at monopolar electrocautery probe 22. Optionally, cannula shaft portion 30 of monopolar electrocautery probe 22 may include a thermal and electrical insulating exterior layer, e.g., plastic or ceramic, to reduce a transfer of heat from the outer periphery of cannula shaft portion 30 to the surrounding tissue.

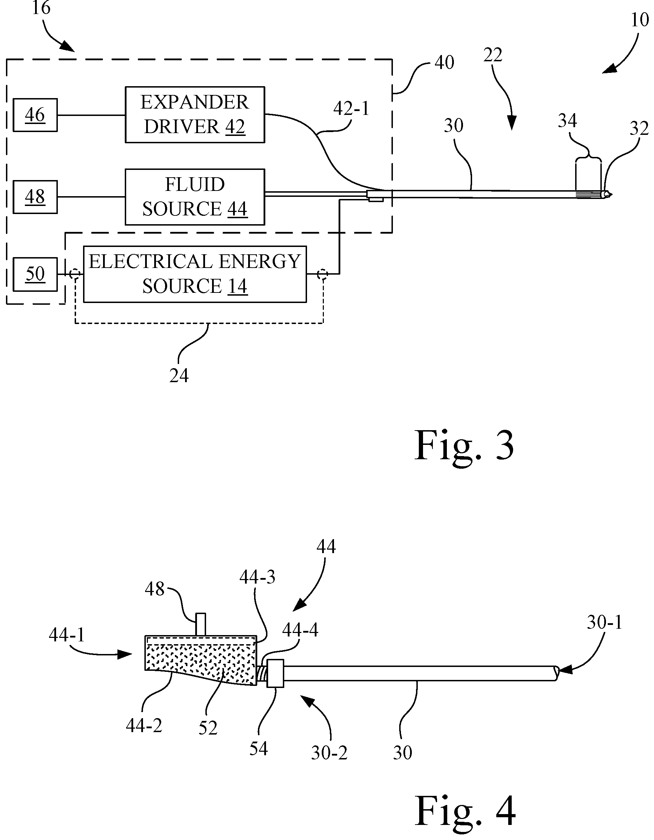

Fig. 2 shows a more detailed view of monopolar electrocautery device 16, which may optionally include an introducer cannula 36, which may be installed coaxial with monopolar electrocautery probe 22 along a longitudinal axis 38. Fig. 3 shows a functional block diagram of system 10, including electrical energy source 14 and monopolar electrocautery device 16 having handpiece 20 and monopolar electrocautery probe 22. Introducer cannula 36 may be made of a biocompatible metal, such as stainless steel, and may include an insulating layer, e.g., plastic or ceramic, on the inner side wall of the introducer cannula 36 to aid in reducing a transfer of heat from the outer periphery of cannula shaft portion 30 to introducer cannula 36. Alternatively, introducer cannula 36 may be made from non-conductive material, such as plastic or ceramic.

Referring to Figs. 2 and 3, handpiece 20 includes a housing 40 that may optionally contain an expander driver 42 and a fluid source 44. Handpiece 20 includes a button 46 for actuating expander driver 42 to deploy monopolar electrocautery probe 22, as will be further explained below. Handpiece 20 also includes a button 48 for actuating fluid source 44 for supplying a sealing fluid to monopolar electrocautery probe 22. Handpiece 20 further includes a button 50 for actuating and controlling the operation of electrical energy source 14 in supplying the RF output signal to monopolar electrocautery probe 22.

In the present embodiment, button 46 may be in the form of a slider member that is slidable along a slot 40-1 formed in housing 40 of handpiece 20. Button 46 is connected to expander driver 42. Expander driver 42 may include a driver member 42-1, such as a push rod or cable, which is mechanically connected to each of, and interposed between, button 46 and distal penetrating tip 32 of monopolar electrocautery probe 22.

Alternatively, expander driver 42 may be an electromechanical device, such as a motor or solenoid, having a linearly movable component that is mechanically connected to driver member 42-1, wherein button 46 serves as a switch to electrically actuate the motor or solenoid of expander driver 42.

Button 48 is connected to fluid source 44 that carries a sealing fluid of a type that is heat-activated. Fluid source 44 is coupled in fluid communication with monopolar electrocautery probe 22, and in particular, cannula shaft portion 30 has a cannula lumen 30-1 that is coupled in fluid communication with fluid source 44. Stated differently, fluid source 44 is configured to deliver the sealing fluid through cannula lumen 30-1 of cannula shaft portion 30 to intermediate portion 34 of monopolar electrocautery probe 22, wherein the sealing fluid may be heat activated by application of heat supplied by monopolar electrocautery probe 22.

Referring also to Fig. 4, in the present embodiment, for example, fluid source 44 is a protein source that carries a protein in a fluid form, wherein the protein is denatured and heat-activated by means of heating monopolar electrocautery probe 22. In other words, the protein source accommodates a substance characterized by a protein which is in the non-denatured or non-heat-activated condition. In the present embodiment, fluid source 44 may be in the form of a syringe 44-1 having a reservoir 44-2 and a piston 44-3 that slidably resides in reservoir 44-2. Reservoir 44-2 is configured as a chamber that contains a sealing fluid 52 (heat-activated) that contains the protein, and button 48 may be a plunger connected to piston 44-3 of syringe 44-1. A Luer fitting 54 is connected to a proximal end 30-2 of cannula shaft portion 30, wherein Luer fitting 54 is in fluid communication with cannula lumen 30-1. Fluid source 44, e.g., syringe 44-1, includes an output port 44-4 that is connected to Luer fitting 54.

Also, in the present embodiment, sealing fluid 52 may be, for example, a solution that contains a protein that is denatured by heat. More particularly, sealing fluid 52 may be, for example, a protein-containing solution that includes a protein, e.g., 10 to 50 % by weight, and optionally may include a crosslinking agent, e.g., 0.1-5.0 % by weight. The protein in the solution may be, for example, albumin. In the optional embodiments that include the crosslinking agent, the crosslinking agent in the solution may be, for example, genipin.

As an alternative to providing fluid source 44 in the form of a syringe, it is contemplated that piston 44-3 of fluid source 44 may be replaced with an electric or pneumatic powered pump, wherein button 48 sends an electrical or pneumatic signal to operate the pump to supply sealing fluid 52 through cannula lumen 30-1 of cannula shaft portion 30 to intermediate portion 34 of monopolar electrocautery probe 22.

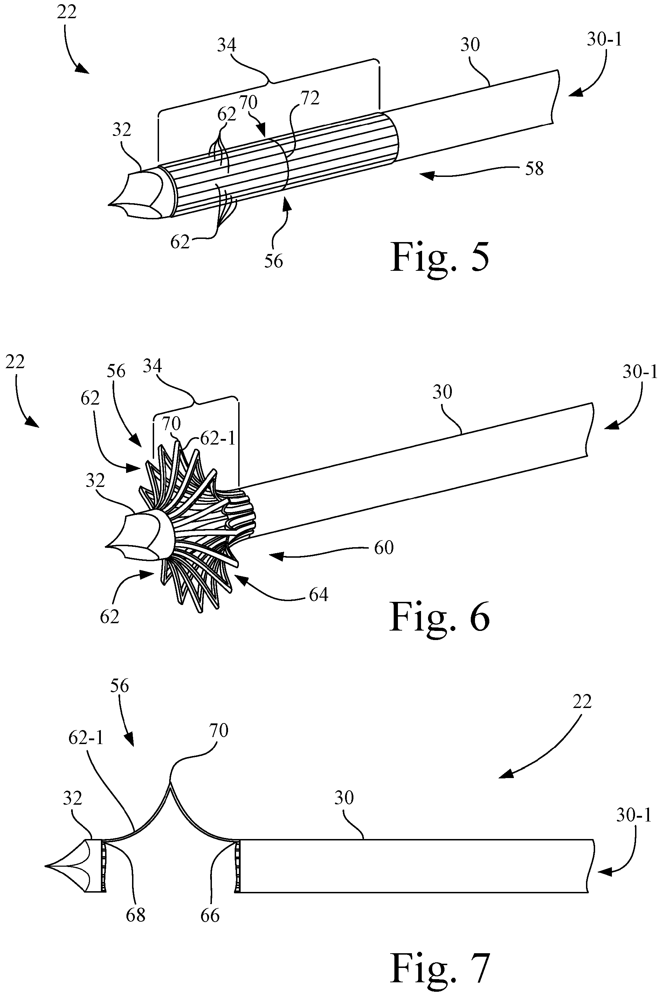

Referring also to Figs. 5 and 6, intermediate portion 34 of monopolar electrocautery probe 22 is configured as an expandable portion 56 having a collapsed state 58 (Fig. 5) and an expanded state 60 (Fig. 6). In particular, the collapsed state 58 of expandable portion 56 is defined by an extended position (see Fig. 5) of distal penetrating tip 32 relative to cannula shaft portion 30, and the expanded state 60 of expandable portion 56 is defined by a retracted position (see Fig. 6) of distal penetrating tip 32 relative to cannula shaft portion 30. Referring also to Fig. 2, in the present embodiment, a transition of state of expandable portion 56 from the collapsed state 58 to the expanded state 60, and vice-versa, may be effected by sliding button 46 along slot 40-1 of housing 40 of handpiece 20. For example, sliding button 46 in a proximal direction along slot 40-1 of housing 40 pulls driver member 42-1 that is attached to distal penetrating tip 32 in the proximal direction, such that the distance between distal penetrating tip 32 and cannula shaft portion 30 at intermediate portion 34 is decreased, thereby expanding expandable portion 56. Optionally, the plurality of expansion members 62 of expandable portion 56 may be formed from a memory material, such as nitinol, so as to aid in the transition from the collapsed state 58 (Fig. 5) to the expanded state 60 (Fig. 6).

It is contemplated that in some embodiments, the use of memory material, e.g., nitinol, for the plurality of expansion members 62 of expandable portion 56, in combination with introducer cannula 36, may be used as a substitute to providing expander driver 42, button 46, and driver member 42-1 connected to distal penetrating tip 32. In such an alternative embodiment, introducer cannula 36 will be slid distally over expandable portion 56 to collapse expandable portion 56 to the collapsed state 58, and introducer cannula 36 will be slid proximally to expose expandable portion 56 such that expandable portion 56 expands in a self-expanding manner to the expanded state 60.

Expandable portion 56 includes a plurality of expansion members 62 at intermediate portion 34. In one embodiment, for example, the plurality of expansion members 62 may be formed by a plurality of longitudinal cuts or slots formed around a periphery of a tubular portion of monopolar electrocautery probe 22 to define intermediate portion 34. In such a case, intermediate portion 34 may be formed from the same material as that of cannula shaft portion 30 of monopolar electrocautery probe 22, such as for example, a biocompatible metal, such as stainless steel.

Alternatively, intermediate portion 34 may be a separate tubular component having a plurality of longitudinal cuts or slots formed around a periphery of a tubular portion of intermediate portion 34, and wherein intermediate portion 34 is inserted between, and attached to each of, cannula shaft portion 30 and distal penetrating tip 32. In such a case, intermediate portion 34 may be formed from a different material, e.g., a different biocompatible metal, from that of cannula shaft portion 30, such as for example, nitinol.

The plurality of expansion members 62 longitudinally extend between cannula shaft portion 30 and distal penetrating tip 32. Also, the plurality of expansion members 62 form an annular periphery of intermediate portion 34 between cannula shaft portion 30 and distal penetrating tip 32.

Expandable portion 56 at intermediate portion 34 is coupled in fluid communication with fluid source 44 via cannula lumen 30-1. Referring to Fig. 6, expandable portion 56 is configured to define a plurality of openings 64, wherein each individual opening of the plurality of openings 64 lies between two adjacent members of the plurality of expansion members 62 around the periphery of intermediate portion 34. Stated differently, a respective opening of the plurality of openings 64 is located between each pair of adjacent expansion members of the plurality of expansion members 62. Accordingly, sealing fluid 52 that is supplied by fluid source 44 (see also Figs. 3 and 4) exits expandable portion 56 through the plurality of openings 64 to a location, e.g., at the pleural layers, external to the monopolar electrocautery probe 22.

Fig. 7 shows an example of an expansion member 62-1 that is representative of each of the plurality of expansion members 62, with the remainder of the individual members of the plurality of expansion members 62 broken away (removed) for clarity. Each expansion member of the plurality of expansion members 62 includes a proximal end 66, a distal end 68, and an articulation joint 70. Proximal end 66 is connected to cannula shaft portion 30, and distal end 68 is connected to distal penetrating tip 32. Articulation joint 70 is located, e.g., half way, between proximal end 66 and distal end 68. Articulation joint 70 may be formed, for example, as a fold line 72 (see Fig. 5) in intermediate portion 34.

Referring again also to Fig. 5, in the collapsed state 58, the diameter of cannula shaft portion 30 and the diameter of expandable portion 56 are substantially equal. However, referring to Fig. 6, in the expanded state 60, the diameter of expandable portion 56 at its largest circumference, i.e., at articulation joint 70, is larger than the diameter of cannula shaft portion 30, e.g., 2 to 4 times larger.

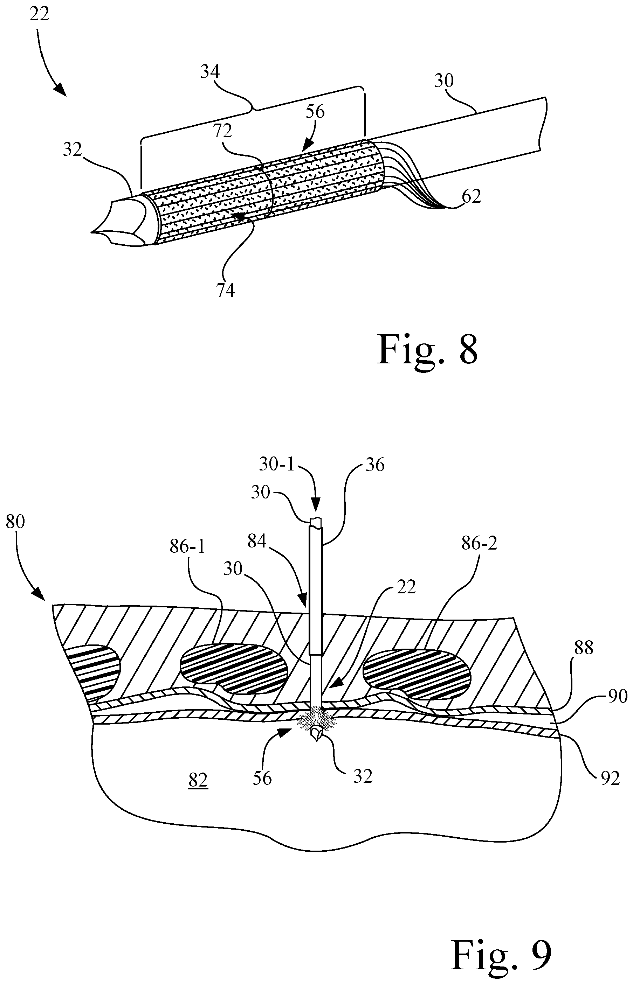

Referring to Fig. 8, as a variation of the previous embodiment, a coating 74 that contains a protein may be applied and formed, e.g., layered, over at least one of the intermediate portion 34 and the distal penetrating tip 32. Coating 74 is configured to be heat-activated, and serves as a protein source that may be a substitute for (not according to the present invention), or supplemental to, fluid source 44. Coating 74 may be formed, in whole or in part, from a protein containing material, such as for example, a material containing collagen. Coating 74 may serve a primary protein source, or alternatively, may serve as a secondary protein source. As a secondary protein source, the collagen may serve as a secondary protein to the primary protein source, e.g., sealing fluid 52.

While in the present embodiment coating 74 is applied over intermediate portion 34 having expandable portion 56 that includes a plurality of expansion members 62, it is contemplated that, alternatively, the coating 74 may be applied to an intermediate portion that does not include expandable portion 56.

Referring to Fig. 9, there is depicted a portion of a chest wall 80 and lung 82 of a patient. Referring again to Fig. 2, monopolar electrocautery probe 22, alone or in combination with introducer cannula 36, may be used to form an access opening 84 to the interior of lung 82. In particular, access opening 84 is formed between adjacent ribs 86-1, 86-2 in the rib cage of chest wall 80, and extends though the parietal pleura 88, the pleural space 90, and the visceral pleura 92 to provide access to the interior of the lung 82. Collectively, parietal pleura 88 and visceral pleura 92 are referred to herein as the pleural layers 88, 92.

Monopolar electrocautery probe 22 is shown positioned in access opening 84, with expandable portion 56 of intermediate portion 34 located distal to (and adjacent), i.e., below, the visceral pleura 92 and in the expanded state 60 (see also Fig. 6). The location of expandable portion 56 of monopolar electrocautery probe 22 may be determined and/or confirmed, using an imaging system, such as for example, ultrasound imaging or X-ray imaging. Fig. 9 shows expandable portion 56 in the expanded state 60 (Fig. 6), so as to aid in compressing the pleural layers 88, 92 when monopolar electrocautery probe 22 is pulled in a proximal direction, toward the user.

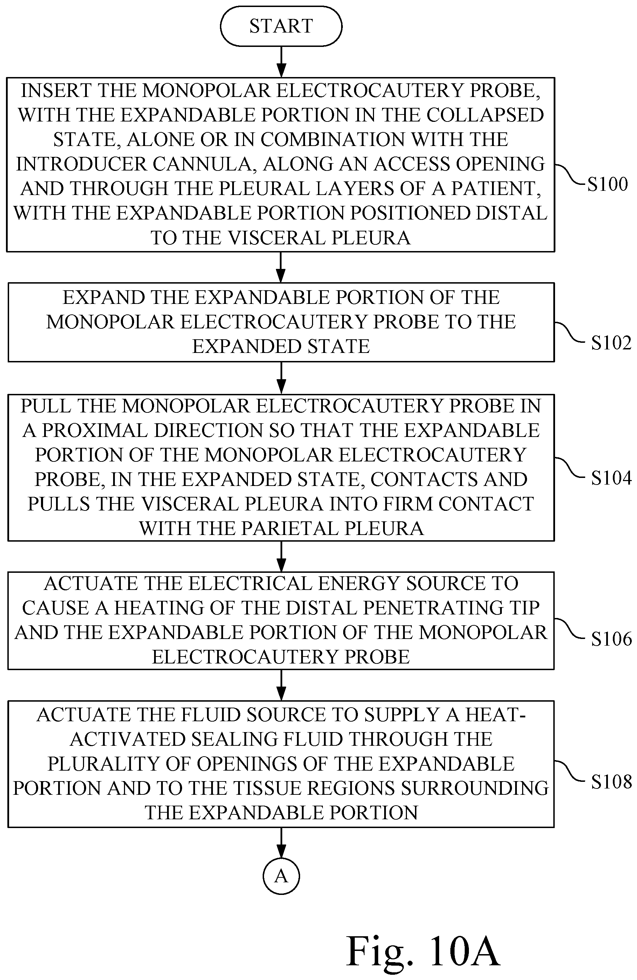

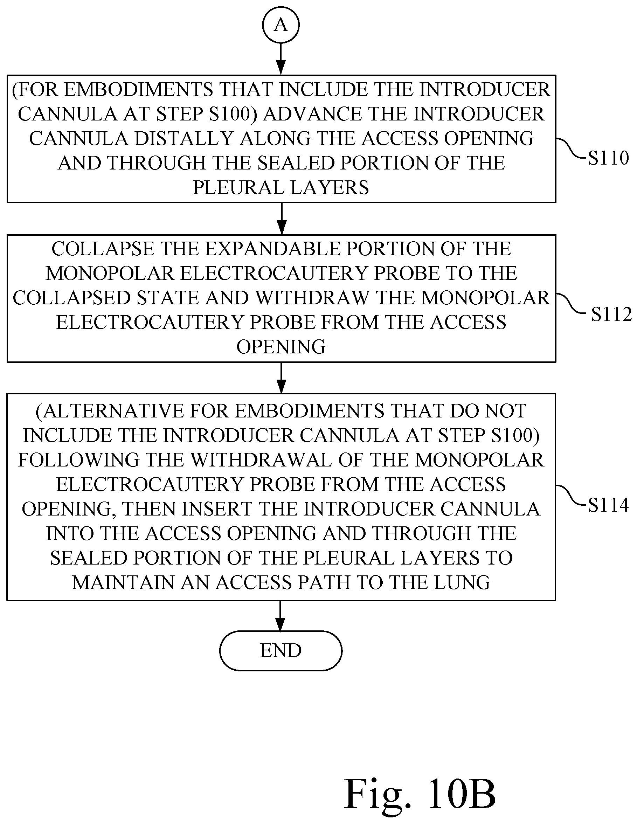

Figs. 10A and 10B depict a flowchart of a method for use in a lung access procedure to aid in preventing pneumothorax. The method will be described, and best understood, with further reference to Figs. 1-6.

At step S100, monopolar electrocautery probe 22 is inserted along access opening 84, with expandable portion 56 of intermediate portion 34 in the collapsed state 58 (see also Figs. 2 and 5), alone or in combination with introducer cannula 36, and through the pleural layers 88, 92 of a patient (see also Fig. 9), with expandable portion 56 of intermediate portion 34 positioned distal to visceral pleura 92.

At step S102, expandable portion 56 of monopolar electrocautery probe 22 is expanded to the expanded state 60 (see also Fig. 6), e.g., by sliding button 46 (see Fig. 2).

At step S104, monopolar electrocautery probe 22 is moved by the user, i.e., pulled, in a proximal direction so that expandable portion 56 of monopolar electrocautery probe 22 (in the expanded state 60; see also Fig. 6) contacts and pulls visceral pleura 92 into firm contact with parietal pleura 88, as depicted in Fig. 9.

At step S106, electrical energy source 14 is actuated, e.g., by depressing button 50 (see Fig. 2) to cause a heating of distal penetrating tip 32 and expandable portion 56 of monopolar electrocautery probe 22.

At step S108, fluid source 44 is actuated, e.g., by depressing button 48 (see Fig. 2) to supply the heat-activated sealing fluid 52 (see Fig. 4) through the plurality of openings 64 of expandable portion 56 (see Fig. 6) and to the tissue regions surrounding expandable portion 56 at step S104, including the pleural layers 88, 92 (see Fig. 9). At this time, with pleural layers 88, 92, being compressed by the prior proximal movement of expandable portion 56, pleural layers 88, 92, are sealed together around access opening 84 by the heat activation of sealing fluid 52.

It is contemplated that steps S106 and S108 may be performed sequentially in the order introduced above, or alternatively, may be performed simultaneously. As a further alternative, it is contemplated the order of performing steps S106 and S 108 may be reversed.

At step S110, in embodiments that include introducer cannula 36 at step S100, introducer cannula 36 may then be advanced distally along access opening 84 and through the sealed portion of the pleural layers 88, 92.

At step S112, expandable portion 56 of monopolar electrocautery probe 22 is collapsed to the collapsed state 58 (see also Fig. 5), e.g., by sliding button 46 (see Fig. 2), and monopolar electrocautery probe 22 may be withdrawn from access opening 84.

At alternative step S114, in embodiments that do not include introducer cannula 36 at step S100, following the withdrawal of monopolar electrocautery probe 22 from access opening 84, then introducer cannula 36 may be inserted into access opening 84 and through the sealed portion of the pleural layers 88, 92 to maintain an access path to lung 82.

Following the positioning of introducer cannula 36 through the sealed portion of the pleural layers 88, 92, a lung procedure, e.g., a lung biopsy, may be performed through introducer cannula 36.

While the primary embodiment above utilizes monopolar electrocautery probe 22, grounding pad 18, and electrical energy source 14 in the form of a radio frequency (RF) generator, it is contemplated that the system may be alternatively (not according to the present invention) be configured to utilize a bipolar electrocautery probe having the structural characteristics as in monopolar electrocautery probe 22 to facilitate localized delivery of the heat-activated protein material. Also, it is contemplated that an electrocautery probe may take other forms, such as an electrocautery probe having an electrical heating element (DC or AC), having the structural characteristics as in monopolar electrocautery probe 22 to facilitate localized delivery of the heat-activated protein material.

As used herein, the term "substantially", and other words of degree, are relative modifiers intended to indicate permissible variation from the characteristic so modified. Such terms are not intended to be limited to the absolute value of the characteristic which it modifies, but rather possessing more of the physical or functional characteristic than the opposite.

While this invention has been described with respect to at least one embodiment, the present invention can be further modified within the scope of this invention as defined by the appended claims. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A system (10) for use in sealing a portion of pleural layers together, comprising:
a fluid source (44) configured to deliver a sealing fluid (52), the sealing fluid being heat-activated;
an electrical energy source (14);
a grounding pad (18) electrically coupled to the electrical energy source (14);
a monopolar electrocautery probe (16) electrically coupled to the electrical energy source (14), wherein the monopolar electrocautery probe (16) and grounding pad (18) cooperate to generate heat,
the monopolar electrocautery probe (16) having a cannula shaft portion (30), a distal penetrating tip (32), and an expandable portion (56) interposed between the cannula shaft portion (30) and distal penetrating tip (32), the cannula shaft portion (30) having a cannula lumen (30-1) coupled in fluid communication with the fluid source (44), **characterized in that** :
the expandable portion (56) is coupled in fluid communication with the fluid source (44) via the cannula lumen (30-1); and
the expandable portion (26) is configured to define a plurality of openings (64), wherein the sealing fluid (52) that is supplied by the fluid source (44) exits the expandable portion (26) through the plurality of openings (64) to a location external to the monopolar electrocautery probe (16).

2. The system according to claim 1, wherein the expandable portion (26) has an extended position that defines a collapsed state and a retracted position that defines an expanded state, the expandable portion including a plurality of expansion members (62), wherein a respective opening of the plurality of openings (64) is located between each pair of adjacent expansion members (62) of the plurality of expansion members.

3. The system according to claim 2, wherein each expansion member (62) of the plurality of expansion members has a proximal end connected to the cannula shaft portion (30) and a distal end connected to the distal penetrating tip (32).

4. The system according to claim 3, wherein each expansion member (62) of the plurality of expansion members further comprises an articulation joint (70) located between the proximal end and the distal end, wherein the expanded state facilitates a flow of the sealing fluid (52) between the plurality of expansion members (62) to a location external to the expandable portion.

5. The system according to any of claims 1 to 4, wherein the cannula shaft portion (30) has a first diameter and the expandable portion (26) has a second diameter, wherein in the collapsed state the first diameter and the second diameter are substantially equal.

6. The system according to any of claims 1 to 4, wherein in the expanded state, the cannula shaft portion (30) has a first diameter and the expandable portion (26) has a largest circumference having a second diameter, wherein the second diameter is greater than the first diameter

7. The system according to any of claims 1 to 6, wherein the expandable portion (26) is made from a biocompatible metal.

8. The system according to any of claims 1 to 7, wherein the fluid source (44) is a syringe.

9. The system according to claim 8, further comprising a Luer fitting connected to a proximal end of the cannula shaft portion (30), the Luer fitting being in fluid communication with the cannula lumen (30-1), and wherein the syringe is connected to the Luer fitting.

10. The system according to any of claims 1 to 9, wherein the sealing fluid (52) includes a protein.

11. The system according to any of claims 1 to 10, comprising a coating (74) formed over at least one of the expandable portion (26) and the distal penetrating tip (32), the coating (74) being a heat-activated material that includes a secondary protein.

## Patentansprüche

1. System (10) zur Verwendung beim Versiegeln eines Teils von pleuralen Schichten miteinander, umfassend:
eine Fluidquelle (44), konfiguriert zum Abgeben eines Versiegelungsfluids (52), wobei das Versiegelungsfluid wärmeaktiviert ist;
eine elektrische Energiequelle (14);
eine Erdungsauflage (18), die mit der elektrischen Energiequelle (14) elektrisch verbunden ist;
eine monopolare Elektrokautersonde (16), die mit der elektrischen Energiequelle (14) elektrisch verbunden ist, wobei die monopolare Elektrokautersonde (16) und die Erdungsauflage (18) zum Erzeugen von Wärme zusammenwirken,
wobei die monopolare Elektrokautersonde (16) einen Kanülenschaftabschnitt (30), eine distale Penetrierspitze (32) und einen ausdehnbaren Abschnitt (56) aufweist, der zwischen dem Kanülenschaftabschnitt (30) und der distalen Penetrierspitze (32) angeordnet ist, wobei der Kanülenschaftabschnitt (30) ein Kanülenlumen (30-1) aufweist, das in Fluidverbindung mit der Fluidquelle (44) verbunden ist, **dadurch gekennzeichnet, dass**:
der ausdehnbare Abschnitt (56) mit der Fluidquelle (44) in Fluidverbindung über das Kanülenlumen (30-1) verbunden ist; und
der ausdehnbare Abschnitt (26) zum Definieren einer Vielzahl von Öffnungen (64) konfiguriert ist, wobei das Versiegelungsfluid (52), das von der Fluidquelle (44) zugeführt wird, aus dem ausdehnbaren Abschnitt (26) durch die Vielzahl von Öffnungen (64) an eine Stelle außerhalb der monopolaren Elektrokautersonde (16) austritt.

2. System nach Anspruch 1, wobei der ausdehnbare Abschnitt (26) eine ausgefahrene Position aufweist, die einen eingeklappten Zustand definiert, und eine eingezogene Position, die einen ausgedehnten Zustand definiert, wobei der ausdehnbare Abschnitt eine Vielzahl von Ausdehnungselementen (62) einschließt, wobei eine jeweilige Öffnung der Vielzahl von Öffnungen (64) zwischen den einzelnen Paaren von benachbarten Ausdehnungselementen (62) der Vielzahl von Ausdehnungselementen angeordnet ist.

3. System nach Anspruch 2, wobei jedes Ausdehnungselement (62) der Vielzahl von Ausdehnungselementen ein proximales Ende aufweist, das mit dem Kanülenschaftabschnitt (30) verbunden ist, und ein distales Ende, das mit der distalen Penetrierspitze (32) verbunden ist.

4. System nach Anspruch 3, wobei jedes Ausdehnungselement (62) der Vielzahl von Ausdehnungselementen weiter eine Gelenkverbindung (70) umfasst, die zwischen dem proximalen Ende und dem distalen Ende angeordnet ist, wobei der ausgedehnte Zustand einen Durchfluss des Versiegelungsfluids (52) zwischen der Vielzahl von Ausdehnungselementen (62) zu einer Stelle außerhalb des ausdehnbaren Abschnitts erleichtert.

5. System nach einem der Ansprüche 1 bis 4, wobei der Kanülenschaftabschnitt (30) einen ersten Durchmesser aufweist und der ausdehnbare Abschnitt (26) einen zweiten Durchmesser aufweist, wobei in dem eingeklappten Zustand der erste Durchmesser und der zweite Durchmesser im Wesentlichen gleich sind.

6. System nach einem der Ansprüche 1 bis 4, wobei in dem ausgedehnten Zustand der Kanülenschaftabschnitt (30) einen ersten Durchmesser aufweist und der ausdehnbare Abschnitt (26) einen größten Umfang mit einem zweiten Durchmesser aufweist, wobei der zweite Durchmesser größer als der erste Durchmesser ist

7. System nach einem der Ansprüche 1 bis 6, wobei der ausdehnbare Abschnitt (26) aus einem biokompatiblen Metall besteht.

8. System nach einem der Ansprüche 1 bis 7, wobei die Fluidquelle (44) eine Spritze ist.

9. System nach Anspruch 8, weiter umfassend ein Luer-Fitting, das mit einem proximalen Ende des Kanülenschaftabschnitts (30) verbunden ist, wobei das Luer-Fitting in Fluidverbindung mit dem Kanülenlumen (30-1) steht, und wobei die Spritze mit dem Luer-Fitting verbunden ist.

10. System nach einem der Ansprüche 1 bis 9, wobei das Versiegelungsfluid (52) ein Protein einschließt.

11. System nach einem der Ansprüche 1 bis 10, umfassend eine Beschichtung (74), die über mindestens eines des ausdehnbaren Abschnitts (26) und der distalen Penetrierspitze (32) gebildet ist, wobei die Beschichtung (74) ein wärmeaktiviertes Material ist, das ein sekundäres Protein einschließt.

## Revendications

1. Système (10) destiné à être utilisé pour sceller une partie de couches pleurales ensemble, comprenant :
une source (44) fluidique configurée pour délivrer un fluide (52) d'étanchéité, le fluide d'étanchéité étant activé par la chaleur ;
une source (14) d'énergie électrique ;
un bornier (18) de mise à la terre couplé électriquement à la source (14) d'énergie électrique ;
une sonde (16) d'électrocautérisation monopolaire couplée électriquement à la source (14) d'énergie électrique, dans lequel la sonde (16) d'électrocautérisation monopolaire et le bornier (18) de mise à la terre coopèrent pour générer de la chaleur,
la sonde (16) d'électrocautérisation monopolaire ayant une partie (30) tige de canule, une pointe (32) pénétrante distale et une partie (56) expansible interposée entre la partie (30) tige de canule et la pointe (32) pénétrante distale, la partie (30) tige de canule ayant une lumière (30-1) de canule couplée en communication fluidique avec la source (44) fluidique, **caractérisé en ce que**
la partie (56) expansible est couplée en communication fluidique avec la source (44) fluidique via la lumière (30-1) de canule ; et
la partie (26) expansible est configurée pour définir une pluralité d'ouvertures (64), dans lequel le fluide (52) d'étanchéité qui est fourni par la source (44) fluidique sort de la partie (26) expansible à travers la pluralité d'ouvertures (64) vers un emplacement externe à la sonde (16) d'électrocautérisation monopolaire.

2. Système selon la revendication 1, dans lequel la partie (26) expansible présente une position étendue qui définit un état affaissé et une position rétractée qui définit un état expansé, la partie expansible incluant une pluralité d'organes (62) d'expansion, dans lequel une ouverture respective de la pluralité d'ouvertures (64) est située entre chaque paire d'organes (62) d'expansion adjacents de la pluralité d'organes d'expansion.

3. Système selon la revendication 2, dans lequel chaque organe (62) d'expansion de la pluralité d'organes d'expansion présente une extrémité proximale connectée à la partie (30) tige de canule et une extrémité distale connectée à la pointe (32) pénétrante distale.

4. Système selon la revendication 3, dans lequel chaque organe (62) d'expansion de la pluralité d'organes d'expansion comprend en outre un joint (70) d'articulation situé entre l'extrémité proximale et l'extrémité distale, dans lequel l'état expansé facilite un flux du fluide (52) d'étanchéité entre la pluralité d'organes (62) d'expansion vers un emplacement externe à la partie expansible.

5. Système selon l'une quelconque des revendications 1 à 4, dans lequel la partie (30) tige de canule a un premier diamètre et la partie (26) expansible a un second diamètre, dans lequel, à l'état affaissé, le premier diamètre et le second diamètre sont sensiblement égaux.

6. Système selon l'une quelconque des revendications 1 à 4, dans lequel, à l'état expansé, la partie (30) tige de canule a un premier diamètre et la partie (26) expansible a une circonférence la plus grande ayant un second diamètre, dans lequel le second diamètre est supérieur au premier diamètre.

7. Système selon l'une quelconque des revendications 1 à 6, dans lequel la partie (26) expansible est réalisée à partir d'un métal biocompatible.

8. Système selon l'une quelconque des revendications 1 à 7, dans lequel la source (44) fluidique est une seringue.

9. Système selon la revendication 8, comprenant en outre un raccord Luer connecté à une extrémité proximale de la partie (30) tige de canule, le raccord Luer étant en communication fluidique avec la lumière (30-1) de canule, et dans lequel la seringue est connectée au raccord Luer.

10. Système selon l'une quelconque des revendications 1 à 9, dans lequel le fluide (52) d'étanchéité inclut une protéine.

11. Système selon l'une quelconque des revendications 1 à 10, comprenant un revêtement (74) formé sur au moins l'une de la partie (26) expansible et de la pointe (32) pénétrante distale, le revêtement (74) étant un matériau activé par la chaleur qui inclut une protéine secondaire.
